# EUROPÄISCHE PATENTSCHRIFT

(11) **EP 3 098 215 B1**
(45) Veröffentlichungstag und Bekanntmachung des Hinweises auf die Patenterteilung: **01.07.2020**
(21) Anmeldenummer: 15169891.7
(22) Anmeldetag: 29.05.2015
(51) Int. Cl.: C07C 51/42, C07C 51/47, C07C 63/26

(54) **VERFAHREN UND VORRICHTUNG ZUM BEHANDELN VON TEREPHTALSÄURE**
METHOD AND DEVICE FOR TREATING TEREPHTHALIC ACID
DISPOSITIF ET PROCEDE DE TRAITEMENT D'ACIDE TEREPHTALIQUE

(43) Veröffentlichungstag der Anmeldung: 30.11.2016
(73) Patentinhaber: BOKELA Ingenieurgesellschaft für Mechanische Verfahrenstechnik mbH, 76131 Karlsruhe (DE)
(72) Erfinder: Bott, Reinhard, 76337 Waldbronn (DE); Schießl, Martin, 75045 Walzbachtal-Wössingen (DE); Neumaier, Klaus, 76131 Karlsruhe (DE)
(74) Vertreter: Wunderlich & Heim Patentanwälte Partnerschaftsgesellschaft mbB

(56) Entgegenhaltungen:
- WO-A2-01/55075
- DE-A1-102012 007 675
- US-A1- 2003 004 373
- John Doe: "RPF Rotary Pressure Filter High process versatility in filtration technology", , 1 November 2014 (2014-11-01), pages 1-16, XP55490058, Sonthofen Retrieved from the Internet: URL:https://www.bhs-sonthofen.de/en/produc ts/filtration-technology/rotary-pressure-f ilter.html [retrieved on 2018-07-04]

## Beschreibung

Die Erfindung betrifft zum einen ein Verfahren zum Behandeln von TA (Terephtalsäure), bei welchem eine Suspension, welche TA-Kristalle und eine Mutterflüssigkeit aufweist, mittels eines Trommelfilters filtriert wird, wobei Mutterflüssigkeit als Filtrat abgeführt wird und die TA-Kristalle sich auf einer Filterfläche des Trommelfilters als ein Haufwerk von TA-Kristallen ablagern, gemäß dem Oberbegriff des Anspruchs 1.

Bei einer Vorrichtung zum Behandeln von TA (Terephtalsäure) ist ein Trommelfilter vorgesehen , welcher eine drehend angetriebene trommelförmige Filterfläche aufweist, die einen Trog durchläuft, welcher mit einer Suspension gefüllt ist, welche TA-Kristalle und Mutterflüssigkeit aufweist, wobei auf der Filterfläche ein Haufwerk von TA-Kristallen gebildet wird.

Terephtalsäure, auch TA ("terephtalic acid") genannt, ist in ihrem gereinigten Zustand, welche häufig als PTA ("purified terephtalic acid") bezeichnet wird, ein wichtiges Ausgangsprodukt in der polymerverarbeitenden Industrie. TA wird im Allgemeinen durch katalytische Oxydation von Paraxylol erhalten. Dabei handelt es sich um eine Reaktionsabfolge mit mehreren Zwischenprodukten. Nach Kristallisation und Entfernung des Polymerisationsprodukts aus dem Oxidationsprozess wird es einem Reinigungsverfahren unterzogen. Dabei wird zunächst das noch unreine Produkt, welches auch CTA ("crude terephtalic acid") bezeichnet wird, in einer geeigneten Löse- oder Mutterflüssigkeit aufgelöst.

Es ist bekannt, das TA in Kristallisationsstufen auszufällen und die TA-Kristalle mittels einer Fest-Flüssig-Trennung von der Reaktionsflüssigkeit zu trennen. Die abfiltrierte Reaktionsflüssigkeit enthält einen großen Teil der Verunreinigungen in gelöster Phase. Zur weiteren Absenkung des Gehalts an Verunreinigungen im TA werden die Kristalle einer Waschung mit Wasser oder anderen Waschflüssigkeiten mit oder ohne Gegenstromführung unterworfen.

Ein derartiges Verfahren und eine derartige Vorrichtung sind beispielsweise aus der DE 10 2012 007 675 A1 bekannt.

Bei der katalytischen Oxidation von Paraxylol kommt häufig ein Kobalt-Katalysator zum Einsatz. Hierdurch ergibt sich eine Verunreinigung der kristallisierten TA mit einer Kobaltsubstanz. Es ist bekannt, diese Kobalt-Verunreinigung unter Verwendung von Essigsäure aus der Terephtalsäure zu entfernen. Nach diesem Reinigungsprozess liegen die TA-Kristalle in einer Mutterflüssigkeit vor, welche zwar frei von Kobalt ist, aber Essigsäure enthält. Essigsäure beeinträchtigt jedoch die weitere Verarbeitung der TA-Kristalle. Es ist daher erforderlich und grundsätzlich auch bekannt, die Essigsäure nach der katalytischen Oxidation von Paraxylol wieder aus der TA zu entfernen.

Gemäß dem Stand der Technik ist es bekannt, Essigsäure aus TA durch einen thermischen Trocknungsprozess zu entfernen. Bei einer thermischen Trocknung von TA wird Essigsäure verdampft und als Gas aus dem Prozess entfernt, so dass eine Beeinträchtigung bei einer nachfolgenden Weiterverarbeitung ausgeschlossen ist. Allerdings ist ein thermisches Trocknen einer Suspension basierend auf TA-Kristallen und einer mit Essigsäure kontaminierten Mutterflüssigkeit energieaufwändig und somit kostenintensiv. Zudem stellt ein thermischer Trocknungsprozess auch eine nicht unerhebliche zeitliche Verzögerung beim Verfahren zum Behandeln von TA dar.

Der Erfindung liegt die **Aufgabe** zugrunde, ein Verfahren anzugeben, mit welchem TA besonders effizient und kostengünstig behandelt werden kann.

Die Aufgabe wird nach der Erfindung durch ein Verfahren mit den Merkmalen des Anspruchs 1 gelöst. Bevorzugte Ausführungsformen der Erfindung sind in den jeweils abhängigen Ansprüchen angegeben.

Das erfindungsgemäße Verfahren ist dadurch gekennzeichnet, dass eine Oberseite des Haufwerks von TA-Kristallen auf der Filterfläche mit einem Behandlungsgas beaufschlagt wird, dass das Behandlungsgas aufgrund einer anliegenden ersten Druckdifferenz das Haufwerk von TA-Kristallen von der Oberseite zu einer Filterfläche durchströmt, wobei verbliebene Mutterflüssigkeit aus dem Haufwerk zur Filterfläche ausgetrieben und das Haufwerk entfeuchtet wird, und dass das entfeuchtete Haufwerk von TA-Kristallen auf der Filterfläche mit einer Waschflüssigkeit beaufschlagt und gewaschen wird, wobei die Waschflüssigkeit aufgrund einer anliegenden zweiten Druckdifferenz den Filterkuchen durchströmt.

Eine Grundidee der Erfindung kann darin gesehen werden, bei der Behandlung von TA zum Entfernen von Mutterflüssigkeit mit einer Kontaminierung, insbesondere von Essigsäure, nach den Kristallisationsstufen keine thermische Trocknung vorzusehen. Vielmehr wird die kontaminierte Mutterflüssigkeit in effizienter Weise durch eine Filtration mit nachfolgender Entfeuchtung und Waschung entfernt. Hierdurch kann in einem erheblichen Maße Energie eingespart werden, welche ansonsten für ein Erhitzen und Verdampfen bei einer thermischen Trocknung notwendig wäre.

Ein zweiter Aspekt der Erfindung liegt darin, dass zum Entfeuchten eine relativ dünne Schicht mit einem lockeren, aber stabilen Haufwerk von TA-Kristallen gebildet wird, indem das Haufwerk durch Anfiltrieren von TA-Kristallen an einer Filterfläche erzeugt wird. Bei der Filtration lassen sich Schichtdicke und Haufwerksstruktur besonders genau einstellen. Zudem wird das anfiltrierte Haufwerk unmittelbar auf der Filterfläche mit einem Behandlungsgas beaufschlagt, durch welches im Wesentlichen ein mechanisches Entfeuchten des Haufwerks aus TA-Kristallen bewirkt wird. Dabei durchdringt das Behandlungsgas aufgrund einer anliegenden ersten Druckdifferenz das Haufwerk auf breiter Front. Die Mutterflüssigkeit, welche insbesondere unerwünschte Essigsäure aufweist, kann nach der Erfindung so als Filtrat zur Filterfläche hin ausgetrieben und über eine Filterzelle abgeführt werden. Die ausgetriebene Mutterflüssigkeit kann dabei gegebenenfalls nach einem Reinigungs- oder Wiederaufbereitungsschritt wieder dem Produktionsprozess zugeführt werden. Ein Anfiltrieren der TA-Kristalle auf der Filterfläche eines Filtermediums, etwa eines Filtergewebes, einer Filtermembran etc., sorgt für einen zuverlässigen hydraulischen Schluss zwischen den mit Mutterflüssigkeit gefüllten Kapillaren im Haufwerk und den Poren im Filterelement. Dies unterstützt ein zuverlässiges mechanisches Entfeuchten.

Ein weiterer Aspekt der Erfindung besteht darin, das so entfeuchtete Haufwerk von TA-Kristallen noch auf derselben Filterfläche mindestens einmal zu waschen, um so eventuell noch verbliebene kontaminierte Mutterflüssigkeit zuverlässig aus dem Haufwerk von TA-Kristallen zu entfernen. Gegebenenfalls kann nach der ersten Waschung eine weitere Entfeuchtung und ein weiteres Waschen mit einer Waschflüssigkeit erfolgen.

Eine bevorzugte Ausführungsform des erfindungsgemäßen Verfahrens besteht darin, dass das Entfeuchten des Behandlungsgases und das Waschen mittels der Waschflüssigkeit mehrfach nacheinander durchgeführt werden. So können an dem Trommelfilter abwechselnd mehrere Entfeuchtungszonen und Waschzonen angeordnet sein. Bevorzugt kann eine drei- oder viermalige Wiederholung des Entfeuchtungs- und Waschzyklus erfolgen. Hierdurch kann ein besonders zuverlässiges Entfernen von Mutterflüssigkeit aus dem Haufwerk von TA-Kristallen sichergestellt werden.

Als Behandlungsfluid kann grundsätzlich jedes Gas eingesetzt werden, aus Explosionsschutzgründen sind jedoch inerte Gase zu bevorzugen. Eine besonders zweckmäßige Verfahrensvariante liegt nach der Erfindung darin, dass als Behandlungsfluid ein Dampf verwendet wird, welcher eine Temperatur aufweist, welche höher als die Temperatur des Haufwerks ist. Unter Dampf wird im Sinne der Erfindung eine gasförmige Phase einer oder mehrerer Flüssigkeiten oder ein Gemisch einer gasförmigen Phase mit Luft oder einem Inertgas verstanden. Vorzugsweise ist der Dampf ein Wasserdampf. Die Temperatur des Dampfes wird dabei in einer bevorzugten Verfahrensausführung in Abhängigkeit der Druckdifferenz so eingestellt, dass der Dampf im Haufwerk aus TA-Kristallen oder im unmittelbaren Anschluss kondensiert und die Kondensationsfront gleichmäßig und etwa parallel zur Filterfläche das Haufwerk zur Filterfläche hin durchwandert. Durch die Verwendung eines kondensierenden Dampfes wird aufgrund der Energiefreigabe bei der Kondensation gezielt Wärmeenergie in das Haufwerk aus TA-Kristallen eingebracht. Die Temperaturerhöhung bewirkt eine Herabsetzung der Viskosität der verbliebenen Mutterflüssigkeit, so dass sich Mutterflüssigkeit aus den Kapillaren in dem Haufwerk leichter entleert. Vorzugsweise wird das Haufwerk dabei auf die Temperatur des Dampfes erwärmt und die Kondensationsfront verschiebt sich entsprechend der Temperaturerhöhung.

Des Weiteren bewirkt das Ausbilden einer Kondensationsfront, welche im Wesentlichen aus der kondensierten Behandlungsflüssigkeit gebildet ist, ein gleichmäßiges Entleeren der Kapillaren, also der mit Mutterflüssigkeit gefüllten Freiräumen zwischen den TA-Kristallen des Haufwerks, weitgehend unabhängig von den Durchmessern der einzelnen Kapillaren, da zwischen zwei Flüssigkeiten keine merkliche Phasengrenze besteht. Gleichzeitig ist in der Kondensationsfront in einem rückwärtigen Bereich zwischen dem gasförmigen Dampf und der kondensierten Flüssigkeit aufgrund beständig ablaufender Kondensations- und Verdampfungsvorgänge keine prägnante Phasengrenze ausgebildet. Auf diese Weise kann verhindert werden, dass das Behandlungsfluid vorzeitig größere Kapillaren entleert und frühzeitig zur Filterfläche durchbricht. Denn entlang vorzeitig entleerter Grobkapillaren könnte sich eine Bypassströmung durch das Haufwerk zur Filterfläche hin ausbilden. Dies würde zu einem erhöhten Verbrauch von Behandlungsgas führen sowie den Energieaufwand zur Aufrechterhaltung der Druckdifferenz erheblich vergrößern oder bei Erreichen der Leistungsgrenze des Kompressors zur Aufrechterhaltung des Druckniveaus sogar unmöglich machen.

Grundsätzlich kann zum Waschen der TA-Kristalle eine beliebige Waschflüssigkeit eingesetzt werden. Besonders wirtschaftlich ist es nach einer weiteren Variante der Erfindung, dass als Waschflüssigkeit Wasser verwendet wird. Dieses steht kostengünstig zur Verfügung und stellt keine Beeinträchtigung für nachfolgende Behandlungsschritte dar.

Das erfindungsgemäße Verfahren kann zum Entfernen einer nahezu beliebigen Mutterflüssigkeit eingesetzt werden. Die Erfindung umfasst vorzugsweise ein Verfahren zum Herstellen von TA, bei welchem durch eine katalytische Oxidation von Paraxylol TA-Kristalle gebildet werden, welche mit der Mutterflüssigkeit gereinigt werden, welche Essigsäure aufweist. Es liegt also eine wirtschaftliche Ausführungsform der Erfindung darin, dass die Mutterflüssigkeit Essigsäure aufweist, durch welche insbesondere Kobalt entfernt wird. Unter Kobalt ist im Sinne der Erfindung jede kobalthaltige Substanz zu verstehen, insbesondere Kobaltsubstanzen, welche bei einer katalytischen Oxidation mit einem Kobaltkatalysator anfallen und in der Mutterflüssigkeit auftreten können.

Grundsätzlich kann das erfindungsgemäße Verfahren auf einem Vakuumfilter oder auf einem sonstigen Drehfilter durchgeführt werden. Eine besonders wirtschaftliche Ausführungsvariante ergibt sich nach der Erfindung dadurch, dass als Trommelfilter ein Druckfilter verwendet wird, welcher ein Druckgehäuse aufweist, in welchem ein Druck an der Oberseite der Filterfläche einstellbar ist. Vorzugsweise wird der über dem zu behandelnden Haufwerk wirkende Differenzdruck dadurch bestimmt oder eingestellt, dass in der Filterzelle mit angeschlossenen Filtratrohren ein Unter-, Atmosphären- oder Überdruck eingestellt wird. Der Trommelfilter weist vorzugsweise eine Anzahl von individuell verrohrten Filterzellen auf. Dadurch kann die Einstellung der Druckdifferenz im Filtrationsbereich, im Entfeuchtungsbereich und/oder im Waschbereich individuell und unterschiedlich eingestellt werden. Dabei kann der Druck in der Filterzelle, auf welcher die Filterfläche aufgespannt ist, Atmosphärendruck, Unter- oder Überdruck betragen. Vorzugsweise kann ein Druck in der Filterzelle eingestellt werden, insbesondere mittels einer Vakuumpumpe ein Unterdruck in der Filterzelle unterhalb der Filterfläche bewirkt werden. Hierdurch ergibt sich eine noch breitere Einstellmöglichkeit der Druckdifferenz.

Eine weitere vorteilhafte Ausführungsform des erfindungsgemäßen Verfahrens besteht darin, dass die Filterfläche zum Bilden des Filterkuchens einen Trog durchläuft, welcher mit Suspension befüllt ist. Die Suspension umfasst dabei die TA-Kristalle und die Mutterflüssigkeit. Grundsätzlich können noch weitere andere Bestandteile in der Suspension enthalten sein. Die drehend angetriebene Filtertrommel durchläuft in einem Filtrationsbereich die Suspension in dem Trog. Aufgrund eines anliegenden Filtrationsdruckes filtrieren die TA-Kristalle zu einem Filterkuchen auf der Filterfläche an. Nach Austritt des so gebildeten Haufwerks aus TA-Kristallen wird dieses vorzugsweise unmittelbar mit dem Behandlungsgas beaufschlagt. Auf diese Weise kann ein Großteil der Mutterflüssigkeit in reiner Form, d.h. ohne Verdünnung mit Waschflüssigkeit, gewonnen werden.

Eine Verunreinigung des entfeuchteten und gewaschenen Haufwerks wird nach einer Weiterbildung der Erfindung dadurch reduziert, dass der Trog gegenüber einem angrenzenden Druckraum des Druckgehäuses abgedeckt wird. Der beckenförmige Trog weist an seiner Oberseite eine plattenförmige Abdeckung auf, welche lediglich einen Durchgang zum Durchtritt der drehenden Filtertrommel mit dem drauf gebildeten Filterkuchen umfasst. Hierdurch wird einer Verdunstung kontaminierter Mutterflüssigkeit entgegengewirkt, welche zu einer Kontamination des bereits gewaschenen Filterkuchens führen könnte, wenn dieser mit der verunreinigten Gasphase in Kontakt kommt.

Das erfindungsgemäße Verfahren ist weiterhin in bevorzugter Weise dadurch weitergebildet, dass das gewaschene Haufwerk von TA-Kristallen in einem feuchten, insbesondere mit Waschflüssigkeit gesättigten Zustand von der Filterfläche abgenommen wird. Bei dieser Verfahrensvariante wird also der Trommelfilter nicht nur zur Fest-Flüssig-Trennung eingesetzt, sondern vielmehr wird die ursprüngliche Mutterflüssigkeit im Filterkuchen durch eine Waschflüssigkeit ersetzt. Dies reduziert grundsätzlich den Filtrations- und Waschungsaufwand. Zudem können die gewaschenen TA-Kristalle, insbesondere bei Verwendung von Wasser als Waschflüssigkeit, in einem feuchten Zustand einer weiteren Verarbeitung zugeführt werden. Üblicherweise wird bei den weiteren Verarbeitungsschritten grundsätzlich Wasser benötigt oder zugesetzt.

Eine Vorrichtung für das Verfahren ist dadurch gekennzeichnet, dass ein Druckgehäuse vorgesehen ist, welches zumindest eine Entfeuchtungskammer, in welcher das anfiltrierte Haufwerk von TA-Kristallen zum Entfeuchten mit einem Behandlungsgas beaufschlagbar ist, und zumindest eine Waschkammer aufweist, in welcher das entfeuchtete Haufwerk von TA-Kristallen zum Waschen mit einer Waschflüssigkeit beaufschlagbar und waschbar ist. Die Vorrichtung kann insbesondere zum Behandeln von TA nach einem Verfahren eingesetzt werden, wie es zuvor beschrieben wurde. Mit der Vorrichtung können so die entsprechend dargelegten Vorteile erzielt werden. Bei einem Trommelfilter im Sinne der Erfindung kann die Filterfläche fest oder als ein ablösbares Band an der Filtertrommel angeordnet sein.

Gemäß einer Weiterbildung der Vorrichtung ist es vorgesehen, dass die Vorrichtung als ein Druckfilter mit einer Druckerzeugungseinrichtung ausgebildet ist, mit welcher in dem Druckgehäuse zumindest bereichsweise ein Überdruck einstellbar ist, welcher über einem Atmosphärendruck liegt. Die Druckerzeugungseinrichtung kann insbesondere ein Kompressor sein. Grundsätzlich können in einem Filtrationsbereich, einem Entfeuchtungsbereich und einem Waschbereich unterschiedliche Druckdifferenzen eingestellt werden. Die Druckdifferenz beträgt vorzugsweise zwischen 1,5 bar bis 15 bar. Abweichende Drücke sind nicht ausgeschlossen.

Eine weitere vorteilhafte Ausführungsvariante der Vorrichtung besteht darin, dass zum Entfeuchten des Haufwerks von TA-Kristallen auf der Filterfläche über einen Steuerkopf ein Überdruck an einer freien Oberseite des anfiltrierten Haufwerks oder eine Entlüftung der unteren Filterzelle erzeugbar ist, auf welcher das zu entfeuchtende Haufwerk auf der Filterfläche gebildet ist. Über einen grundsätzlich bekannten Steuerkopf an einem Trommelfilter kann bereichsweise im Druckgehäuse und insbesondere in den entsprechenden Unterkammern des Druckgehäuses ein definierter Differenzdruck eingestellt werden, welcher auf die freie Oberseite des anfiltrierten Haufwerks wirkt. Hierdurch wird vorzugsweise die Druckdifferenz in der jeweiligen Zone eingestellt. Alternativ oder ergänzend kann die jeweilige Filterzelle, welche unter der Filterfläche liegt und von den in Umfangsrichtung angrenzenden Filterzellen druckdicht abgeteilt ist, eine Entlüftung oder auch ein Überdruck erzeugt werden. Auch hierdurch kann die jeweilige Druckdifferenz in den einzelnen Zonen eingestellt werden.

Nach einer weiteren Ausführungsform ist es vorteilhaft, dass eine Druckgaseinheit vorgesehen ist, mit welcher das Haufwerk von TA-Kristallen von der Filterfläche von unten von einer Filterzelle mittels eines Druckgasstoßes entfernbar ist und dass eine Spüleinrichtung vorgesehen ist, mit welcher die Filterzelle vor einem Erzeugen des Druckgasstoßes spülbar ist. Vorzugsweise wird die Filterzelle vor einem Druckgasstoß zum Lösen und Entfernen des Haufwerks von TA-Kristallen von der Filterfläche intern mit einer Waschflüssigkeit gespült. Hierdurch wird sichergestellt, dass sich in der Filterzelle keine Reste mehr von kontaminierter Mutterflüssigkeit befinden, welche wieder eine Verunreinigung in das Haufwerk eintragen könnten. Die Waschflüssigkeit ist vorzugsweise dieselbe Waschflüssigkeit, mit der das Haufwerk auf der Filterfläche gespült wird. Insbesondere wird zum Spülen der Filterzelle Wasser verwendet.

Der Druckgasstoß kann in bekannter Weise durch eine Druckgaseinheit, etwa einen Kompressor oder einen Druckgasbehälter oder einen Dampfgenerator, erzeugt werden. Durch den Druckgasstoß wird das Haufwerk von unten von der Filterfläche abgeblasen. Das Druckgas kann ein beliebiges Gas, insbesondere ein Inertgas oder Luft, oder ein Dampf sein.

Nach einer Weiterbildung ist vorgesehen, dass die Filterzelle vor Abnahme des anfiltrierten Haufwerks zum Entleeren von Flüssigkeit einen Belüftungssteuerkopf, der vorzugsweise auf der Gegenseite des eigentlichen Steuerkopfes angeordnet ist, oder eine Belüftungsbohrung im eigentlichen Steuerkopf aufweist. Hierdurch kann bei einem nachfolgenden Abblasen des Filterkuchens sichergestellt werden, dass keine kontaminierte Flüssigkeit aus den Filtratrohren wieder in den Filterkuchen gelangt.

Die Erfindung umfasst weiterhin eine Anlage zum Herstellen von TA, welche eine Oxidationsvorrichtung, in welcher Paraxylol oxidiert wird und TA-Kristalle gebildet werden, und eine Reinigungsvorrichtung aufweist, in welcher den TA-Kristallen eine Mutterflüssigkeit zugeführt wird, wobei die vorbeschriebene Vorrichtung zum Behandeln von TA vorgesehen ist. Mit der Anlage können auch das vorbeschriebene Verfahren durchgeführt und die genannten Vorteile erzielt werden.

Die Erfindung wird nachfolgend anhand von bevorzugten Ausführungsbeispielen weiter beschrieben, welche schematisch in den beigefügten Zeichnungen dargestellt sind. In den Zeichnungen zeigen:
- Fig. 1: eine schematische Seitenansicht zu einer ersten Ausführungsform einer Vorrichtung für das erfindungsgemäße Verfahren;
- Fig. 2: eine perspektivische Ansicht zu einer zweiten Ausführungsform einer Vorrichtung für das erfindungsgemäße Verfahren;
- Fig. 3: eine teilweise geschnittene Darstellung einer Filtertrommel zu einer dritten Ausführungsform einer Vorrichtung;
- Fig. 4: eine schematische Querschnittsansicht der Filtertrommel von Fig. 3 gemäß einer ersten Betriebsweise;
- Fig. 5: eine schematische Querschnittsansicht der Filtertrommel gemäß Fig. 3 gemäß einer zweiten Betriebsweise;
- Fig. 6: eine schematische Seitenansicht zu einer vierten Ausführungsform einer Vorrichtung;
- Fig. 7: eine schematische Seitenansicht zu einer fünften Ausführungsform einer Vorrichtung;
- Fig. 8: eine schematische Seitenansicht zu einer sechsten Ausführungsform einer Vorrichtung;
- Fig. 9: eine schematische Seitenansicht zu einer siebten Ausführungsform einer Vorrichtung; und
- Fig. 10: eine schematische Seitenansicht zu einer achten Ausführungsform einer Vorrichtung.

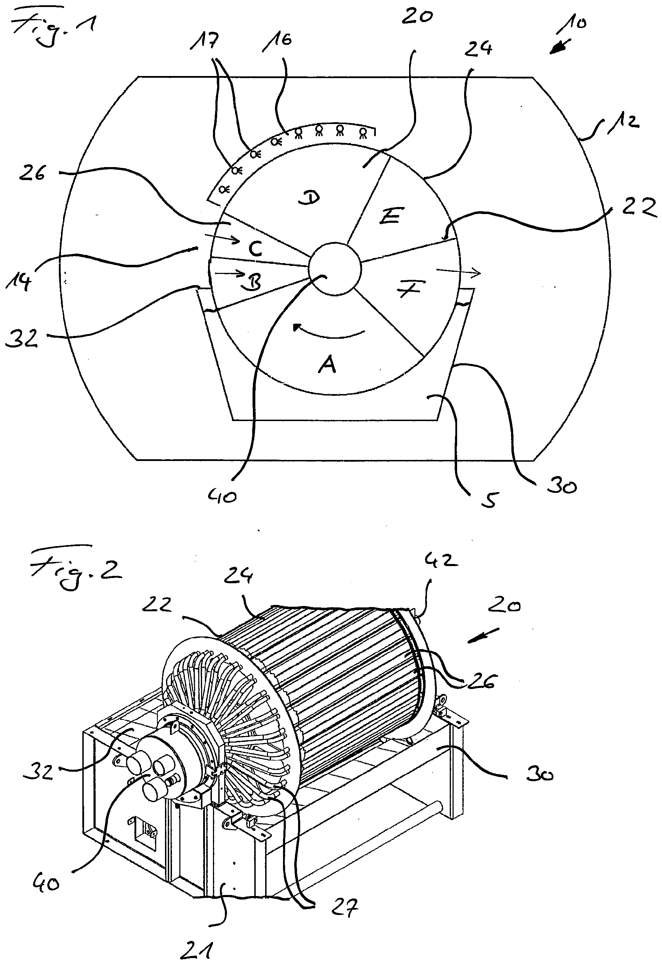

Das erfindungsgemäße Verfahren wird im Zusammenhang mit der schematischen Darstellung einer ersten Ausführungsform einer Vorrichtung 10 gemäß Fig. 1 erläutert. Die Vorrichtung 10 zum Behandeln von TA weist einen Trommelfilter 20 mit einer um eine horizontale Drehachse drehbar gelagerten Filtertrommel 22 auf. Die Filtertrommel 22 ist in bekannter Weise aus einer Vielzahl von im Querschnitt kreissegmentförmigen Filterzellen 26 aufgebaut, welche über einen zentralen Steuerkopf 40 zur Druckeinstellung angesteuert werden.

Die Filtertrommel 22 ist drehend im Uhrzeigersinn angetrieben, wie durch einen Pfeil in Fig. 1 dargestellt ist. Dabei durchläuft die Filtertrommel 22 einen Trog 30, welcher mit einer Suspension 5 gefüllt ist. Die Suspension 5 umfasst eine Mutterflüssigkeit mit TA-Kristallen. Die Mutterflüssigkeit kann dabei einen Anteil Essigsäure enthalten oder insgesamt aus Essigsäure bestehen. Mutterflüssigkeit mit Essigsäure kann bei der katalytischen Oxidation von TA-Kristallen zu dessen Reinigung eingesetzt werden.

Der Trommelfilter 20 ist in einem druckdichten Druckgehäuse 12 angeordnet, welches mit einem Behandlungsgas befüllt ist. Um einen übermäßigen Austritt von verdampfter Mutterflüssigkeit aus dem Trog 30 zu verhindern, weist der Trog 30 an seiner Oberseite eine plattenartige Abdeckung 32 auf.

Zur Durchführung des erfindungsgemäßen Verfahrens durchläuft der zylindrische Trommelfilter 22, an dessen trommelförmiger Umfangswand eine Filterfläche 24 durch ein Filtertuch oder eine Filtermembran gebildet ist, innerhalb des Troges 30 eine Kuchenbildungszone A. Aufgrund einer anliegenden Druckdifferenz, welche über den Steuerkopf 40 gesteuert wird, wird Suspension 5 angesaugt, wobei Mutterflüssigkeit durch die Filterfläche 24 hindurchtritt und als Filtrat über den Steuerkopf 40 abgeführt wird. Gleichzeitig lagern sich TA-Kristalle an der Filterfläche 24 als ein Haufwerk an. Die Dicke des Haufwerkes kann bis zu einigen Zentimetern betragen. Nach Austritt aus der Suspension 5 und dem Trog 30 durchläuft die Filtertrommel 22 mit dem anfiltrierten Haufwerk eine erste Zellenentfeuchtungszone B. Dabei strömt ein Behandlungsgas, welches unter einem vorgegebenen Druck in dem Druckgehäuse 12 ist, aufgrund einer anliegenden ersten Druckdifferenz in das Haufwerk aus TA-Kristallen von der Oberseite in Richtung auf die Filterfläche 24 ein. Das Behandlungsgas verdrängt dabei im Haufwerk verbliebene Mutterflüssigkeit und treibt dieses zur Filterfläche 24 hin aus. Dabei wird das Haufwerk aus TA-Kristallen entfeuchtet. Der Bereich vor einer Waschzone D stellt eine Entfeuchtungskammer 14 dar.

Anschließend durchläuft das entfeuchtete Haufwerk mit der Filterzelle 26 die sich anschließende erste Zellentleerungszone C, bei welcher die Filterzelle 26 insgesamt von Filtrat, also von der Mutterflüssigkeit, entleert wird, indem die Mutterflüssigkeit über den Steuerkopf 40 aus dem Trommelfilter 20 abgeführt wird. Anschließend erfolgt ein Durchlauf durch eine erste Waschzone D, in welcher das entfeuchtete Haufwerk aus TA-Kristallen auf der Filterfläche 24 mit einer Waschflüssigkeit gewaschen wird. Hierzu ist im Bereich der ersten Waschzone D eine haubenförmige Waschkammer 16 vorgesehen, in welcher eine Vielzahl von Waschdüsen 17 angeordnet ist. Über die Waschdüsen 17 wird eine Waschflüssigkeit, vorzugsweise Wasser, auf das entfeuchtete Haufwerk aufgesprüht, so dass dieses wieder befeuchtet und gewaschen wird. Aufgrund einer anliegenden zweiten Druckdifferenz, welche ebenfalls durch den Steuerkopf 40 eingestellt wird, durchströmt die Waschflüssigkeit das Haufwerk aus TA-Kristallen. Dabei wird sichergestellt, dass verbliebene säurehaltige Mutterflüssigkeit zuverlässig aus dem Haufwerk aus TA-Kristallen ausgewaschen wird. Im Anschluss hieran durchläuft die Filterzelle 26 mit dem so gewaschenen Haufwerk eine zweite Entfeuchtungszone E, in welcher aufgrund einer anliegenden dritten Druckdifferenz, welche der ersten Druckdifferenz entsprechen kann, das Haufwerk aus TA-Kristallen von Waschflüssigkeit entfeuchtet werden kann. Dabei strömt das Behandlungsgas aus dem Druckgehäuse 12 aufgrund der anliegenden Druckdifferenz in das Haufwerk von der Oberseite in Richtung auf die Filterfläche 24 in das Haufwerk ein, wobei Waschflüssigkeit durch die Filterfläche 24 nach innen verdrängt und abgeführt wird.

Nach der zweiten Entfeuchtung durchläuft das gewaschene und entfeuchtete Haufwerk eine Abnahmezone F, in welcher das Haufwerk von TA-Kristallen über eine Abnahmeeinrichtung, insbesondere über eine Druckgaseinheit, von der Filterfläche 24 entfernt und abgenommen wird. Nach einer optionalen Reinigung der Filterfläche 24 kann die jeweilige Filterzelle 26 der Filtertrommel 22 für einen erneuten Verfahrenszyklus in die Kuchenbildungszone A in den Trog 30 wieder eintreten.

In Fig. 2 ist eine Ausführungsform eines Trommelfilters 20 schematisch dargestellt, welcher grundsätzlich in der Vorrichtung 10 nach Fig. 1 eingesetzt werden kann. Die etwa zylindrische Filtertrommel 22 ist horizontal drehbar an einem Gestell 21 gelagert, in welchem auch der Trog 30 angeordnet ist. Ein nach oben gerichteter Freiraum zwischen der Filtertrommel 22 und dem äußeren Rand des Troges 30 ist über eine plattenförmige Abdeckung 32 im Wesentlichen dicht abgedeckt. Zur Ansteuerung der einzelnen Filterzellen 26 ist eine Vielzahl von Filtratrohren 27 an der Filtertrommel 22 vorgesehen, welche sich von den Filterzellen 26 zu einem Steuerkopf 40 erstrecken. An der dem Steuerkopf 40 gegenüberliegenden Stirnseite der Filtertrommel 22 ist ein nur schematisch angedeuteter Belüftungssteuerkopf 42 vorgesehen.

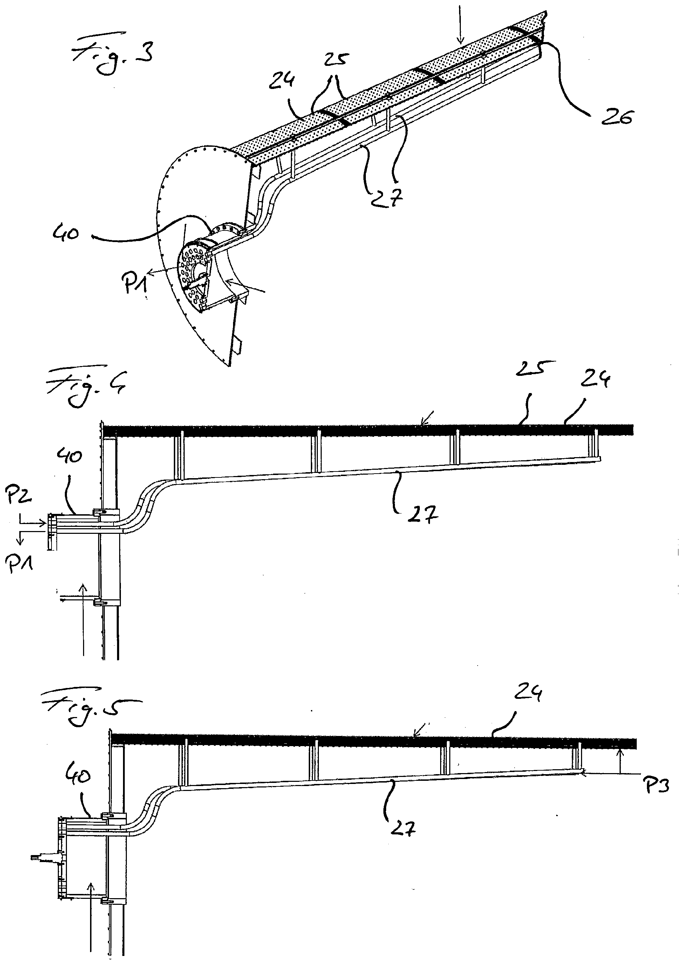

Im Zusammenhang mit den Figuren 3 bis 5 werden die Anordnung der Filtratrohre 27 sowie verschiedene Betriebsweisen anhand weiterer Ausführungsvarianten näher erläutert.

Gemäß Fig. 3 weist eine zylindersegmentförmige Filterzelle 26 an ihrer Außenseite mehrere längliche gebogene Stützplatten 25 auf, welche zum Durchtritt von Filtrat ausgebildet sind. Auf den Stützplatten 25 ist zum Bilden der Filterfläche 24 ein nicht dargestelltes Filtermedium, insbesondere ein Filtergewebe oder eine Filtermembran, angeordnet. An der Unterseite der Stützplatten 25 erstrecken sich Filtratrohre 27, über welche aufgrund einer anliegenden Druckdifferenz Mutterflüssigkeit, welche durch die Filterfläche 24 und die perforierten Stützplatten 25 hindurchgetreten ist, zum Steuerkopf 40 abgeführt wird. Aus dem Steuerkopf 40 tritt das Filtrat entsprechend dem Pfeil P1 aus und kann einer Weiterverarbeitung oder Entsorgung zugeführt werden.

Wie in Fig. 4 dargestellt ist, kann über die Filtratrohre 27 nicht nur Filtrat von der Filterfläche 24 gemäß dem Pfeil P1 aus der Filtertrommel 22 abgeführt werden. Vielmehr kann über die Filtratrohre 27 auch ein Druckgas gemäß dem Pfeil P2 vom Steuerkopf 40 zu der Filterfläche 24 geleitet werden, um etwa Haufwerk von der Filterfläche 24 abzublasen. Da jeweils zwei Filtratrohre 27 mit einer Filterzelle 26 verbunden sind, kann die Entleerung der Filtratrohre 27 und der Filterzelle 26 dadurch unterstützt werden, dass in Richtung des Pfeils P2 Druckgas zugeführt wird, welches verbleibendes Filtrat über das andere Filtratrohr 27 ausbläst. Dabei muss eine Druckdifferenz über dem Filterkuchen erhalten bleiben, damit dieser auf der Filterfläche24 gehalten wird.

Gemäß der weiteren Ausführungsform nach Fig. 5 kann von dem hier nicht gezeigten Belüftungssteuerkopf 42 Gas von der dem Steuerkopf 40 gegenüberliegenden Seite in die Filtratrohre 27 geleitet werden. Auf diese Weise kann ebenfalls ein Gas zum Ablösen des Haufwerks von der Filterfläche 24 durch die Filtratrohre 27 eingeleitet werden. Gleichzeitig können bei dieser Anordnung die Filtratrohre 27 gespült werden, so dass mit dieser Spüleinrichtung ein Entfernen der säurehaltigen Mutterflüssigkeit zuverlässig auch aus den Filtratrohren 27 ermöglicht wird. Diese Anordung kann auch dazu benutzt werden den Austrag von in den Filtratrohren 27 befindlichem Filtrat zu unterstützen. Dabei muss eine Druckdifferenz über dem Filterkuchen erhalten bleiben, damit dieser auf der Filterfläche 24 gehalten wird.

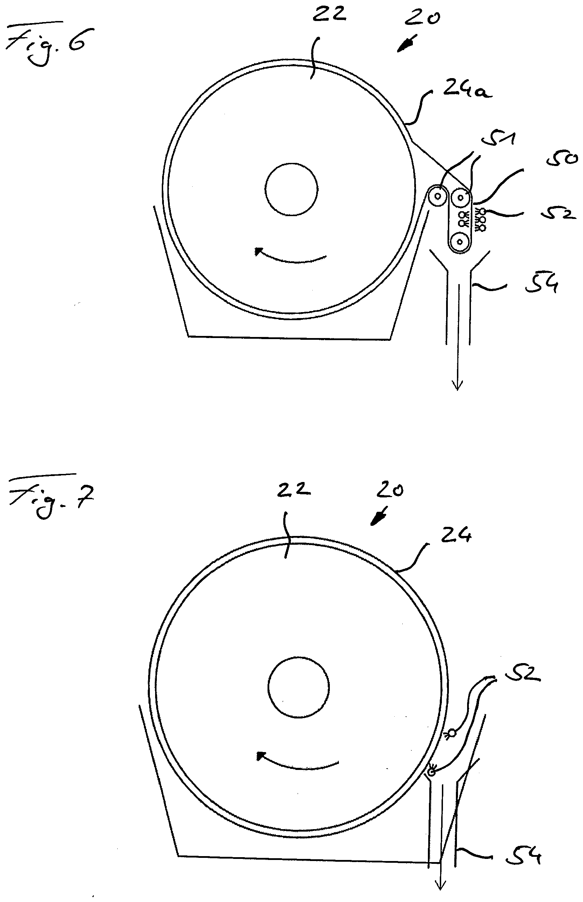

Bei der abgewandelten Ausführungsform nach Fig. 6 weist der Trommelfilter 20 eine Filtertrommel 22 auf, bei welcher eine als umlaufendes Band ausgebildete Filterfläche 24a vorgesehen ist. Bei dieser Ausführungsvariante kann die Filterfläche 24a zum Entfernen des Haufwerks von der Filtertrommel 22 bereichsweise abgelöst und einer Reinigungseinrichtung 50 mit Umlenkrollen 51 zugeführt werden. Dies erleichtert eine Abnahme des Haufwerks von TA-Kristallen von der Filterfläche 24a. Zudem kann die Reinigungseinrichtung 50 mit Spüldüsen 52 versehen sein, mit welchen eine Vorderseite und eine Rückseite der umlaufenden Filterfläche 24a gereinigt werden kann. Das abgenommene Haufwerk und/oder ablaufende Reinigungsflüssigkeit der Spüldüsen 52 kann über einen Abführschacht 54 nach unten abgeführt werden.

Eine weitere abgewandelte Ausführungsform eines Trommelfilters 20 für die Vorrichtung 10 ist in Fig. 7 gezeigt. Die Filterfläche 24 ist dabei als ein Filtertuch oder eine Filtermembran auf der Filtertrommel 22 aufgespannt. Zur Reinigung der Filterfläche 24 von angelagerten TA-Kristallen können Spüldüsen 52 vorgesehen sein, welche auf die Oberfläche der Filterfläche 24 gerichtet sind. Die so gelösten TA-Kristalle können über einen Abführschacht 54 abgeführt werden.

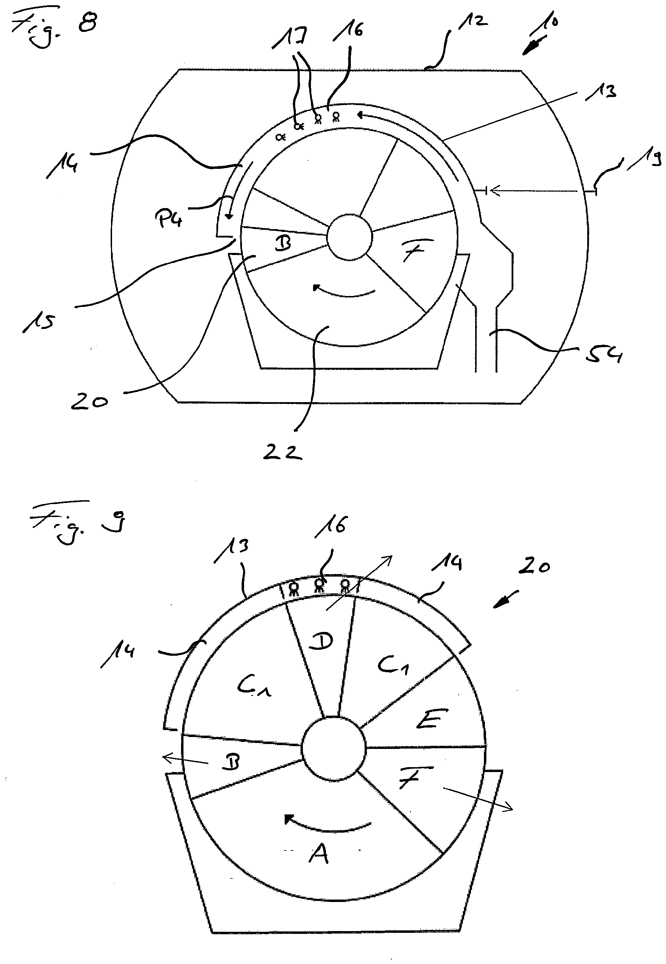

Eine weitere abgewandelte Ausführungsform der Erfindung ist in Fig. 8 dargestellt. In dem Druckgehäuse 12 der Vorrichtung 10 ist ein Trommelfilter 20 angeordnet, welcher grundsätzlich aufgebaut ist, wie der Trommelfilter von Fig. 1. Entlang der freien Oberseite der zylindrischen Filtertrommel 22 erstreckt sich eine Haube 13 durchgehend von der Zellentfeuchtungszone B bis zu einem Abführschacht 54 zum Abführen des Haufwerks aus TA-Kristallen. In der durchgehenden Haube 13 sind die Entfeuchtungskammer 14 sowie die Waschkammer 16 mit den Waschdüsen 17 angeordnet. Weiterhin ist eine Druckgasleitung 19 vorgesehen, welche sich durch das Druckgehäuse 12 zum Inneren der Haube 13 erstreckt und in den Bereich der Abnahmezone F mündet. Aufgrund eines höheren Druckes strömt eingeleitetes Druckgas innerhalb der Haube 13 von der Mündung der Druckgasleitung 19 entgegen der Drehrichtung der Filtertrommel 22 gemäß dem Pfeil P4 zu einer Haubenöffnung 15, an welcher das anfiltrierte Haufwerk in die Haube 13 eintritt. Aufgrund eines Überdruckes innerhalb der Haube 13 gegenüber der Atmosphäre in dem Druckgehäuse 12 tritt so beständig Druckgas aus der Haube 13 auf. Hierdurch wird verhindert, dass verdampfte säurehaltige Mutterflüssigkeit in den Bereich der Haube 13 eindringt und mit dem gewaschenen und entfeuchteten Haufwerk aus TA-Kristallen in einen nachfolgenden Bearbeitungsschritt gelangen kann.

Gemäß der Ausführungsform nach Fig. 9 ist ein Trommelfilter 20 mit einer Haube 13 gezeigt, welche eine Waschkammer 16 aufweist, welche von zwei abgegrenzten Entfeuchtungskammern 14 umgeben ist. Hierdurch sind vor und nach der Waschzone D eine erste Zellentleerungszone C1 beziehungsweise eine zweite Zellentleerungszone C2 gebildet. Bei dieser Anordnung kann in die Entfeuchtungskammern 14 insbesondere ein Dampf als Behandlungsgas eingeleitet werden.

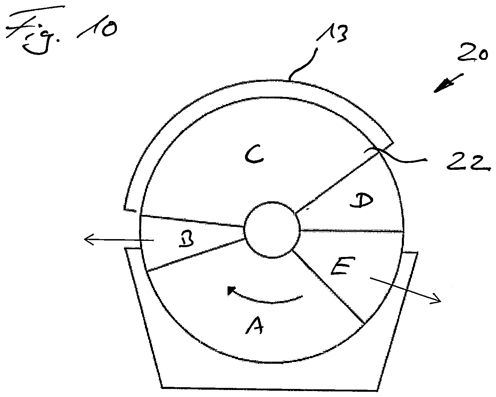

Bei der weiteren Ausführungsvariante gemäß Fig. 10 weist der Trommelfilter 20 eine Haube 13 auf, welche einen Teil des Umfangs der Filtertrommel 22 umgibt. In die Haube 13 kann ausschließlich Behandlungsgas, insbesondere Dampf, eingeleitet werden, um so über einen großen Durchlaufbereich eine Zellentleerungszone C zu bilden.

Die Ausführungsvarianten gemäß den Figuren 6 bis 10 weisen, auch wenn dies nicht näher beschrieben ist, ein Druckgehäuse 12 mit einer entsprechenden Betriebsweise auf, wie sie im Zusammenhang mit Fig. 1 beschrieben worden ist.

## Patentansprüche

1. Verfahren zum Behandeln von TA (Terephtalsäure), bei welchem eine Suspension (5), welche TA-Kristalle und eine Mutterflüssigkeit aufweist, mittels eines Trommelfilters (20) filtriert wird, wobei Mutterflüssigkeit als Filtrat abgeführt wird und die TA-Kristalle sich auf einer Filterfläche (24) des Trommelfilters (20) als ein Haufwerk von TA-Kristallen ablagern, wobei eine Oberseite des Haufwerks von TA-Kristallen auf der Filterfläche (24) mit einem Behandlungsgas beaufschlagt wird, das Behandlungsgas aufgrund einer anliegenden ersten Druckdifferenz das Haufwerk von TA-Kristallen von der Oberseite zu der Filterfläche (24) durchströmt, das entfeuchtete Haufwerk von TA-Kristallen auf der Filterfläche (24) mit einer Waschflüssigkeit beaufschlagt und gewaschen wird, wobei die Waschflüssigkeit aufgrund einer anliegenden zweiten Druckdifferenz das Haufwerk durchströmt,
**dadurch gekennzeichnet,**
- **dass** der Trommelfilter (20) mit Filterzellen (26) bereitgestellt wird,
- **dass** zum Entfeuchten des Haufwerks verbliebene Mutterflüssigkeit aus dem Haufwerk zur Filterfläche (24) ausgetrieben und über die unter der Filterfläche (24) liegenden Filterzelle (26) abgeführt wird,
- **dass** zum Lösen und Entfernen des Haufwerks von der Filterfläche (24) ein Druckgasstoß mittels einer Druckgaseinheit von unten von der Filterzelle (26) vorgesehen wird, wobei das Haufwerk von der Filterfläche (24) abgeblasen wird, und
- **dass** die Filterzelle (26) vor dem Erzeugen des Druckgasstoßes mittels einer Spüleinrichtung intern gespült wird.

2. Verfahren nach Anspruch 1,
**dadurch gekennzeichnet,**
**dass** das Entfeuchten mittels des Behandlungsgases und das Waschen mittels der Waschflüssigkeit mehrfach nacheinander durchgeführt werden.

3. Verfahren nach Anspruch 1 oder 2,
**dadurch gekennzeichnet,**
**dass** als Behandlungsfluid Dampf verwendet wird, welcher eine Temperatur aufweist, welche höher als die Temperatur des Haufwerks ist.

4. Verfahren nach einem der Ansprüche 1 bis 3,
**dadurch gekennzeichnet,**
**dass** als Waschflüssigkeit Wasser verwendet wird.

5. Verfahren nach einem der Ansprüche 1 bis 4,
**dadurch gekennzeichnet,**
**dass** die TA-Kristalle durch eine katalytische Oxidation gebildet werden und dass die TA-Kristalle vor dem Anfiltrieren mit einer Mutterflüssigkeit gereinigt werden, welche Essigsäure aufweist.

6. Verfahren nach einem der Ansprüche 1 bis 5,
**dadurch gekennzeichnet,**
**dass** als Trommelfilter (20) ein Druckfilter verwendet wird, welcher ein Druckgehäuse (12) aufweist, in welchem ein Druck an der Oberseite der Filterfläche (24) einstellbar ist und der über dem zu behandelnden Haufwerk wirkende Differenzdruck dadurch bestimmt wird, dass in der Filterzelle (26) mit angeschlossenen Filtratrohren (27) ein Unter-, Atmosphären- oder Überdruck eingestellt wird.

7. Verfahren nach einem der Ansprüche 1 bis 6,
**dadurch gekennzeichnet,**
**dass** die Filterfläche (24) zum Bilden des Haufwerks einen Trog (30) durchläuft, welcher mit Suspension (5) befüllt ist.

8. Verfahren nach Anspruch 7,
**dadurch gekennzeichnet,**
**dass** der Trog (30) gegenüber einem angrenzenden Gasdruckraum des Druckgehäuses (12) abgedeckt wird.

9. Verfahren nach einem der Ansprüche 1 bis 8,
**dadurch gekennzeichnet,**
**dass** das gewaschene Haufwerk von TA-Kristallen in einem feuchten, insbesondere mit Waschflüssigkeit gesättigten Zustand von der Filterfläche (24) abgenommen wird.

## Claims

1. Method for treating TA (terephthalic acid), in which
a suspension (5) having TA-crystals and a mother liquid is filtered by means of a drum filter (20), wherein mother liquid is discharged as filtrate and the TA-crystals deposit on a filter area (24) of the drum filter (20) as an aggregate of TA-crystals, wherein an upper side of the aggregate of TA-crystals on the filter area (24) is acted upon by a treatment gas, due to an applied first pressure difference the treatment gas flows through the aggregate of TA-crystals from the upper side towards the filter area (24), the dehumidified aggregate of TA-crystals on the filter area (24) is acted upon by a washing liquid and washed, wherein the washing liquid flows through the aggregate due to an applied second pressure difference.
**characterized in that**
- the drum filter (20) is provided with filter cells (26),
- **in that** residual mother liquid is expelled from the aggregate towards the filter area (24) for dehumidifying the aggregate and is discharged via the filter cell (26) positioned under the filter area (24),
- **in that** for releasing and discharging the aggregate from the filter area (24) a pressure gas impact is provided by means of a pressure gas unit from bellow of the filter cell (26), wherein the aggregate is blown off from the filter area (24), and
- **in that** the filter cell (26) is internally flushed prior to the provition of the pressure gas impact by means of a flushing device.

2. Method according to claim 1,
**characterized in that**
the dehumidification by means of the treatment gas and the washing by means of the washing liquid are carried out several times in succession.

3. Method according to claim 1 or 2,
**characterized in that**
as treatment fluid steam is used which has a temperature that is higher than the temperature of the aggregate.

4. Method according to any one of claims 1 to 3,
**characterized in that**
as washing liquid water is used.

5. Method according to any one of claims 1 to 4,
**characterized in that**
the TA-crystals are formed through catalytic oxidation and
**in that** prior to the filtration the TA-crystals are purified with a mother liquid having acetic acid.

6. Method according to any one of claims 1 to 5,
**characterized in that**
as drum filter (20) a pressure filter is used which has a pressure housing (12), in which a pressure on the upper side of the filter area (24) can be set and the differential pressure acting above the aggregate to be treated is determined **in that** in the filter cell (26) with connected filtrate pipes (27) an under-, atmospheric or overpressure is set.

7. Method according to any one of claims 1 to 6,
**characterized in that**
for the formation of the aggregate the filter area (24) runs through a trough (30) which is filled with suspension (5).

8. Method according to claim 7,
**characterized in that**
the trough (30) is covered with respect to an adjoining gas pressure compartment of the pressure housing (12).

9. Method according to any one of claims 1 to 8,
**characterized in that**
the washed aggregate of TA-crystals is removed from the filter area (24) in a humid state, in particular in a state of being saturated with washing liquid.

## Revendications

1. Procédé de traitement d'acide téréphtalique, où
une suspension (5), laquelle présente des cristaux d'acide téréphtalique et une liqueur mère, est filtrée au moyen d'un filtre à tambour (20), dans lequel de la liqueur mère est évacuée en tant que filtrat et les cristaux d'acide téréphtalique s'accumulent sur une surface filtrante (24) du filtre à tambour (20) en tant qu'un agrégat de cristaux d'acide téréphtalique, dans lequel un côté supérieur de l'agrégat de cristaux d'acide téréphtalique sur la surface filtrante (24) est soumis à l'action d'un gaz de traitement, le gaz de traitement traverse, en raison d'une première pression différentielle appliquée, l'agrégat de cristaux d'acide téréphtalique depuis le côté supérieur vers la surface filtrante (24), l'agrégat déshumidifié de cristaux d'acide téréphtalique sur la surface filtrante (24) est soumis à l'action d'un liquide de lavage et est lavé, dans lequel le liquide de lavage traverse l'agrégat en raison d'une deuxième pression différentielle appliquée,
**caractérisé en ce**
- **que** le filtre à tambour (20) est fourni avec des cellules filtrantes (26),
- **que** pour déshumidifier l'agrégat, de la liqueur mère résiduelle est chassée de l'agrégat vers la surface filtrante (24) et est évacuée par l'intermédiaire de la cellule filtrante (26) située sous la surface filtrante (24),
- **que** pour défaire et éliminer l'agrégat de la surface filtrante (24), une salve de gaz sous pression est prévue au moyen d'une unité de gaz sous pression depuis le bas depuis la cellule filtrante (26), dans lequel l'agrégat est éliminé par soufflage de la surface filtrante (24), et
- **que** la cellule filtrante (26) est rincée côté interne au moyen d'un dispositif de rinçage avant la production de la salve de gaz sous pression.

2. Procédé selon la revendication 1,
**caractérisé en ce**
**que** la déshumidification est mise en œuvre au moyen du gaz de traitement et le lavage au moyen du liquide de lavage est mis en œuvre à plusieurs reprises l'un après l'autre.

3. Procédé selon la revendication 1 ou 2,
**caractérisé en ce qu'**est utilisée en tant que fluide de traitement de la vapeur, laquelle présente une température, laquelle est plus élevée que la température de l'agrégat.

4. Procédé selon l'une quelconque des revendications 1 à 3,
**caractérisé en ce**
**que** de l'eau est utilisée en tant que liquide de lavage.

5. Procédé selon l'une quelconque des revendications 1 à 4,
**caractérisé en ce que** les cristaux d'acide téréphtalique sont obtenus par une oxydation catalytique, et que les cristaux d'acide téréphtalique sont nettoyés avec une liqueur mère avant le filtrage, laquelle présente de l'acide acétique.

6. Procédé selon l'une quelconque des revendications 1 à 5,
**caractérisé en ce**
**qu'**est utilisé en tant que filtre à tambour (20) un filtre sous pression, lequel présente un boîtier sous pression (12), dans lequel une pression peut être réglée au niveau du côté supérieur de la surface filtrante (24) et la pression différentielle agissant par l'intermédiaire de l'agrégat à traiter est définie **en ce qu'**une dépression, une pression atmosphérique ou une surpression sont réglées dans la cellule filtrante (26) avec des tubes de filtrat (27) raccordés.

7. Procédé selon l'une quelconque des revendications 1 à 6,
**caractérisé en ce**
**que** la surface filtrante (24), pour obtenir l'agrégat, traverse une auge (30), laquelle est remplie de suspension (5).

8. Procédé selon la revendication 7,
**caractérisé en ce**
**que** l'auge (30) est recouverte par rapport à un espace à pression de gaz adjacent du boîtier sous pression (12).

9. Procédé selon l'une quelconque des revendications 1 à 8,
**caractérisé en ce**
**que** l'agrégat lavé de cristaux d'acide téréphtalique est retiré de la surface filtrante (24) dans un état humide, en particulier saturé de liquide de lavage.
